(19) Europäisches Patentamt
European Patent Office
Office européen des brevets

(11) Veröffentlichungsnummer: **0 152 868**
**B1**

# (12) EUROPÄISCHE PATENTSCHRIFT

(45) Veröffentlichungstag der Patentschrift: **21.06.89**

(51) Int. Cl.⁴: **C 07 D 261/18**, C 07 D 413/12, A 61 K 31/445, A 61 K 31/42

(21) Anmeldenummer: **85101264.1**

(22) Anmeldetag: **07.02.85**

## (54) Isoxazol-Derivate, Verfahren zu ihrer Herstellung und diese Verbindungen enthaltende Arzneimittel.

(30) Priorität: **17.02.84 DE 3405727**

(43) Veröffentlichungstag der Anmeldung: **28.08.85 Patentblatt 85/35**

(45) Bekanntmachung des Hinweises auf die Patenterteilung: **21.06.89 Patentblatt 89/25**

(84) Benannte Vertragsstaaten: **AT BE CH DE FR GB IT LI LU NL SE**

(56) Entgegenhaltungen:
**DE-A-2 524 959**
**DE-C-634 286**
**DE-C-653 835**

(73) Patentinhaber: **HOECHST AKTIENGESELLSCHAFT, Postfach 80 03 20, D-6230 Frankfurt am Main 80 (DE)**

(72) Erfinder: **Kämmerer, Friedrich- Johannes, Dr., Am Gänsborn 3a, D-6203 Hochheim am Main (DE)**
Erfinder: **Schleyerbach, Rudolf, Dr., Finkenweg 10, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 152 868 B1

LIBER, STOCKHOLM 1989

**Beschreibung**

Die Erfindung betrifft neue 4-Isoxazolcarbonsäureamide sekundärer cyclischer Amine, Verfahren zu deren Herstellung und diese Verbindungen enthaltende Arzneimittel, die sich insbesondere zur Behandlung von Schmerz- und Fieberzuständen eignen, bzw. die Verwendung der Verbindungen zur Behandlung von Schmerz- und Fieberzuständen.

Zu den üblichen weltweit verwendeten schwachen oder milden Analgetika, die gegen die starken morphinähnlichen Analgetika mit ihrer mehr oder weniger stark ausgeprägten Fähigkeit zur Erzeugung von Sucht und Gewöhnung abzugrenzen sind und daher häufig auch als "nicht-opioide" Analgetika bezeichnet werden, zählen die Salicylate, Pyrazolone und das Anilin-Derivat Paracetamol (Acetaminophen). Die mit ihnen gemachten therapeutischen Erfahrungen zeigen, daß sie wirksam sind und ein vertretbares Nutzen-Risikoverhältnis aufweisen. Dennoch ist ersichtlich, daß es kein schwaches Analgetikum ohne mehr oder weniger gravierende Nebenwirkungen zum Teil sehr spezifischer Art gibt.

Dazu gehört beispielsweise die zwar äußerst selten auftretende, aber mitunter tödlich verlaufende Agranulocytose nach Pyrazolon-Einnahme. Paracetamol ist in normalen therapeutischen Dosen sicher, bei Überdosierung jedoch hepatotoxisch. Unter Acetylsalicylsäure-Medikation kommt es häufig zu Magen-Darmschädigungen, wie okkulten Blutungen in der Schleimhaut, Magangeschwüren und Perforation vorhandener peptischer Ulcera; ein weiterer Nachteil besteht in der hohen Wechelwirkungsrate mit anderen Medikamenten.

Es besteht daher ein dringendes Bedürfnis nach Analgetika, die diese unerwünschten Nebenwirkungen nicht besitzen.

Überraschend wurde nun gefunden, daß man mit der Herstellung von 4-Isoxazolcarbonsäureamiden sekundärer cyclischer Amine zu einer Verbindungsklasse gelangt, deren Vertreter hervorragende analgetische und antipyretische Eigenschaften ohne gastrale Nebenwirkungen bei guter Leberverträglichkeit in chronischen Toxizitätsversuchen besitzen.

Im Gegensatz zu den meisten bekannten schwachen Analgetika weisen die erfindungsgemäßen Verbindungen bemerkenswerterweise keine antiphlogistische Wirkungskomponente auf. Die Ursache hierfür liegt darin, daß die erfindungsgemäßen Verbindungen keinen hemmenden Einfluß auf die periphere Prostaglandin-Biosynthese haben. Hierin ist zugleich der Grund für ihre gute Magenverträglichkeit zu suchen, denn bei dem analgetisch wirksamen Antiphlogistika werden nicht nur der therapeutisch genutzte entzündungshemmende Effekt, sondern auch die unerwünschten gastrointestinalen Nebenwirkungen auf eben diese Inhibierung der Prostaglandin-Synthetasen im peripheren Gewebe zurückgeführt.

Die neuen 4-Isoxazolcarbonsäureamide stellen folglich antipyretisch aktive Analgetika ohne antiphlogistischen Effekt dar, die von ihrem Wirkungsprofil her dem klinisch etablierten Paracetamol (vergl. G. Kuschinsky und H. Lüllmann, Kurzes Lehrbuch der Pharmakologie, 6. überarbeitete und erweiterte Auflage, Georg Thieme Verlag Stuttgart 1974, S. 109 ff) an die Seite zu stellen sind.

Aus der deutschen Patentschrift 634 286 ist ein Verfahren zur Herstellung von Isoxazolcarbonsäureamiden bekannt, die wertvolle therapeutische Eigenschaften aufweisen und deshalb als Heilmittel verwendet werden sollen. Eine Indikationsangabe findet sich in dieser Patentschrift micht. Die strukturell den erfindungsgemäßen Verbindungen nächstvergleichbaren Verfahrensprodukte der Beispiele 5 und 6 sind jedenfalls, wie festgestellt wurde, analgetisch unwirksam.

In der deutschen Patentschrift 653 835, die einen Zusatz zum Patent 634 286 darstellt, wird den Verbindungen des Hauptpatents eine analeptische Wirkung zugeschrieben.

Ferner sind aus der deutschen Patentschrift 2 524 959 4-Isoxazol-carbonsäureanilide mit antiphlogistischen und analgetischen Eigenschaften bekannt. Bei diesen Verbindungen steht jedoch die antiphlogistische Wirkung ganz eindeutig im Vordergrund, die auch hier im wesentlichen auf eine Hemmung der peripheren Prostaglandin-Biosynthese zurückzuführen ist.

Gegenstand der Erfindung sind demgegenüber Verfahren zur Herstellung von Verbindungen der Formel (I) (s. Patentanspruch 1), worin

R Wasserstoff, Alkyl mit bis zu 4 C-Atomen oder Halogenalkyl mit bis zu 2 C-Atomen und
W eine direkte Bindung, $CH_2$, $CH-CH_3$, $CH-C_2H_5$, CHOH, O oder S bedeuten.

Bevorzugt sind dabei solche Verbindungen, in denen R Methyl, Äthyl oder Trifluormethyl darstellt. Unter diesen Verbindungen sind wiederum diejenigen besonders bevorzugt, in denen W für $CH_2$ steht.

Bei der folgenden Beschreibung der Verfahren haben R und W die vorstehend genannten Bedeutungen. Ein Verfahren besteht darin, daß man das sekundäre cyclische Amin der Formel II (s. Patentanspruch 1) mit einem 4-Isoxazolcarbonsäure-Derivat der Formel III (s. Patentanspruch 1) umsetzt, in der X entweder ein Halogenatom, vorzugsweise Chlor oder Brom, oder eine YO- oder ZO-CO-O-Gruppe bedeutet, wobei Y für Phenyl, durch Fluor, Chlor, Brom, Jod, Methyl, Äthyl, Methoxy, Äthoxy, Trifluormethyl, Nitro oder Cyan einfach, zweifach oder dreifach substituiertes Phenyl, oder für den Acylrest der allgemeinen Formel IIIa (s. Patentanspruch 1), und Z für $(C_1-C_4)$-Alkyl, Phenyl oder Benzyl stehen.

Die Reaktion wird zweckmäßig in einem Verteilungs- oder Lösemittel durchgeführt, das sich unter den Reaktionsbedingungen gegenüber den Reaktionspartnern indifferent verhält. Hierfür kommen beispielsweise Nitrile wie Acetonitril, Äther wie Diäthyläther, Tetrahydrofuran, Dioxan, Alkohole wie Methanol, Äthanol,

Propanol oder Isopropanol, und Wasser in Frage.

In einer bevorzugten Ausführungsform wird das Amin der Formel II mit dem betreffenden Carbonsäurechlorid der Formel III, zweckmäßig in Gegenwart eines säurebindenden Mittels wie Kalium- oder Natriumcarbonat, Alkali- oder Erdalkalihydroxid oder -alkoholat, einer organischen Base, wie Triäthylamin, Pyridin, Picolin oder Chinolin oder des im Überschuß eingesetzten Amins der Formel (II), im allgemeinen bei Temperaturen von 0 bis 120°C, vorzugsweise von 20 bis 60°C, umgesetzt. Die Reaktionszeiten können von wenigen Minuten bis zu zwei Stunden betragen.

Sofern man in organischen Lösemitteln arbeitet, werden die Verfahrensprodukte der Formel I vorteilhaft nach Abfiltrieren der als Nebenprodukte ausgefallenen Salze durch Einengen der Filtrate isoliert. Aus wäßrigen Reaktionsmischungen lassen sich die Produkte vorteilhaft durch Extraktion mit einem polaren organischen Lösemittel wie Methylenchlorid, Chloroform oder Trichloräthylen und Eindampfen der Extrakte gewinnen. Die Produkte können anschließend durch Destillation oder Umkristallisieren aus einem organischen, vorzugsweise mäßig polaren Lösemittel wie Toluol, Dimethylbenzol, Benzol, Cyclohexan, Methanol, Äthanol, Diäthyl- oder Diisopropyläther oder unpolaren Solventien, wie Petroläther oder aber einem Gemisch aus solchen Lösemitteln gereinigt werden.

Die 4-Isoxazolcarbonsäure-Derivate der Formel III können nach üblichen Methoden aus den entsprechenden Carbonsäuren hergestellt werden. Die hierfür als Ausgangsstoffe benötigten 4-Isoxazolcarbonsäuren (Formel III: X = OH) sind bekannt (Deutsche Patentschrift 634 286, Europäische Patentanmeldung 12 435 und Gazz. Chim. Ital. 96 (4), 443 - 453 (1966)) oder aber analog darstellbar.

Ein weiteres Verfahren zur Herstellung von Verbindungen der Formel I ist dadurch gekennzeichnet, daß man ein 2-Acylessigsäureamid der Formel IV (s. Patentanspruch 1) mit einem Orthoameisensäureester der Formel $HC(OR^1)_3$ (V), worin $R^1$ $(C_1\text{-}C_4)$Alkyl bedeutet, umsetzt und das dabei erhaltene 2-Alkoxymethylen-2-acylessigsäureamid der Formel VI (s. Patentanspruch 1) mit Hydroxylamin cyclisiert.

Hierbei kann beispielsweise so vorgegangen werden, daß man das Amid der Formel IV mit einer zweckmäßig mindestens äquimolaren Menge eines Orthoameisensäureesters der Formel V, gegebenenfalls in Gegenwart eines Alkohol-bindenden Mittels, auf eine Temperatur von 80 bis 150°C, vorzugsweise auf die Siedetemperatur des Gemisches, erwärmt, das so erhaltene 2-Alkoxymethylen-2-acylessigsäureamid der Formel VI isoliert und anschließend mit einer zweckmäßig mindestens äquimolaren Menge Hydroxylamin in einem organischen Lösemittel oder Lösemittelgemisch, vorzugsweise in einem mit Wasser mischbaren cyclischen Äther wie Tetrahydrofuran oder Dioxan, gegebenenfalls unter Zusatz von bis zu 2 Vol.-Teilen, vorzugsweise bis zu 1 Vol.-Teil, Wasser auf 1 Vol.-Teil organisches Lösemittel, im allgemeinen bei einer Temperatur von 0 bis 130°C, vorzugsweise von 20 bis 100°C umsetzt. Die Reaktionszeiten liegen meistens zwischen wenigen Minuten und etwa 5 stunden.

Eine weitere bequeme Darstellungsmethode für die Isoxazole der Formel I besteht in der Umsetzung der 2-Acylessigsäureamide der Formel IV mit sekundären Aminen der Formel $HNR^2R^3$ (VII) (s. Patentanspruch 4), in der $R^2$ und $R^3$ gleiche oder verschiedene Alkylreste mit 1 bis 4 C-Atomen oder zusammen mit dem N-Atom einen Ring wie Pyrrolidin, Piperidin oder Morpholin darstellen, zu den Enaminen der Formel VIII (s. patentanspruch 1), und deren Cyclisierung mit dem aus Nitromethan intermediär erzeugten Nitriloxid vorteilhaft in Gegenwart wasserentziehender Mittel.

Die Synthese der als Zwischenprodukte benötigten Enamine VIII kann in der Weise erfolgen, daß man das Acylessigsäureamid der Formel IV mit einer zweckmäßig mindestens äquimolaren Menge des sekundären Amins der Formel VII, vor teilhaft unter Zusatz einer katalytischen Menge Säure, wie Ameisensäure oder p-Toluolsulfonsäure, zweckmäßig in einem Verteilungs- oder Lösemittel, das sich unter den Reaktionsbedingungen gegenüber den Reaktionspartnern indifferent verhält, z. B. einem aromatischen Kohlenwasserstoff wie Toluol, im allgemeinen bei Temperaturen von 60 bis 160°C, vorzugsweise 80 bis 130°C, umsetzt.

Die anschließende Cyclisierungsreaktion des Enamins der Formel VIII mit Nitromethan zum Verfahrensprodukt der Formel I gelingt in üblicher Weise (G. Stork et al., J. Amer. Chem. Soc. 89, 1967, S. 5461 - 5462) durch Umsetzung entweder in Gegenwart eines Isocyanats wie Phenylisocyanat und einer organischen Base, wie etwa Triäthylamin, in einem Kohlenwasserstoff, z. B. Benzol oder Toluol, bei Temperaturen von 0°C bis zur Siedetemperatur des Reaktionsgemisches oder aber unter der Mitwirkung eines anorganischen Säurehalogenids, beispielsweise Phosphoroxychlorid, in einem halogenierten Kohlenwasserstoff, wie etwa Chloroform, wobei vorzugsweise um 0°C gearbeitet wird.

Die erfindungsgemgäßen Isoxazolverbindungen der Formel I können auf Grund ihrer pharmakologischen Eigenschaften als Arzneimittel, insbesondere als Analgetika und Antipyretika, Verwendung finden. Sie können entweder allein, gegebenenfalls in Form von Mikrokapseln, oder vermischt mit geeigneten Trägerstoffen verabreicht werden.

Gegenstand der Erfindung sind somit auch Arzneimittel, die aus einer Verbindung der Formel I bestehen oder diesen Wirkstoff neben einem pharmazeutisch üblichen und physiologisch verträglichen Trägerstoff, Verdünnungsmittel und/oder anderen Hilfsstoffen enthaltan. Diese Mittel können oral, rektal oder parenteral appliziert werden, wobei die orale und rektale Anwendung bevorzugt ist.

Geeignete feste oder flüssige galenische Zubereitungsformen sind beispielsweise Granulate, Pulver, Dragees, Tabletten, Kapseln, Suppositorien, Sirupe, Säfte, Suspensionen, Emulsionen, Tropfen oder injizierbare Lösungen sowie Präparate mit protrahierter Wirkstofffreigabe. Als häufig verwendete Trägermittel seien z. B. Calciumcarbonat, Calciumphosphate, verschiedene Zucker oder Stärkearten, Cellulosederivate,

Gelatine, pflanzliche Öle, Polyäthylenglykole und physiologisch unbedenkliche Lösemittel genannt.

Eine weitere Anwendung der Verbindungen gemäß Formel I besteht in der Kombination mit anderen geeigneten Wirkstoffen, beispielsweise anderen zentral wirksamen Analgetika wie Codein auf das zentrale Nervensystem wirkende Stimuantien wie Coffein oder anderen Spasmolytika.

Vorzugsweise werden die Präparate in Dosierungseinheiten hergestellt und verabreicht, wobei jede Einheit eine bestimmte Dosis an aktiver Substanz gemäß Formel I enthält. Bei festen Dosierungseinheiten wie Tabletten, Kapseln und Suppositorien kann diese Dosis bis zu 1 500 mg, bevorzugt 100 bis 600 mg, und bei Injektionslösungen in Ampullenform bis zu 1000 mg, vorzugsweise 50 bis 500 mg, betragen.

Für die Behandlung eines an Schmerzen und/oder Fieber leidenden erwachsenen Patienten sind - je nach Wirksamkeit der Verbindung gemäß Formel I am Menschen - Tagesdosen von 200 bis 3 000 mg Wirkstoff, vorzugsweise 500 bis 1 000 mg bei oraler oder rektaler Verabreichung und von 100 bis 1 500 mg, bevorzugt 200 bis 600 mg bei intravenöser Applikation indiziert. Bei Kindern kann als Tagesdosis je nach Lebensalter z. B. schon ein Fünftel der vorgenannten unteren Werte ausreichen.

Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Zeitintervallen erfolgen.

**Herstellungsbeispiele**

Die Struktur aller nachstehend beschriebenen Verbindungen wurde durch Elementaranalyse und IR- sowie $^{1}H$-NMR-Spektren gesichert.

1) <u>N-(5-Methyl-4-isoxazolylcarbonyl)-piperidin</u>

a) 0,2 Mol 5-Methyl-4-isoxazolcarbonsäurechlorid (29,1 g), gelöst in 350 ml Acetonitril, werden unter Rühren bei Raumtemperatur tropfenweise mit einer Lösung von 0,4 Mol piperidin (34,1 g) in 50 ml Acetonitril so versetzt, daß die Temperatur der Reaktionslösung nicht über 40°C steigt. Anschließend wird 15 Minuten nachgerührt und dann auf Raumtemperatur abgekühlt. Man saugt das ausgefallene Piperidin-Hydrochlorid ab und engt das Filtrat unter vermindertem Druck ein. Der ölige Rückstand wird in 300 ml Methylenchlorid gelöst und mit 40 ml 2 N Natronlauge gewaschen.

Die Methylenchloridphase wird abgetrennt und die Wasserphase nochmals mit 200 ml Methylenchlorid extrahiert. Die vereinigten Methylenchloridphasen werden mit 70 ml 0,2 N Salzsäure geschüttelt, mit Wasser neutral gewaschen und nach dem Trocknen über Natriumsulfat unter vermindertem Druck zur Trockne gebracht. Der ölige Rückstand wird unter vermindertem Druck destilliert. Man erhält so N-(5-Methyl-4-isoxazolylcarbonyl)-piperidin mit einem Siedepunkt von 102 bis 112°C bei 4 bis 7 mbar (Kugelrohrdestillation), das nach kurzer Zeit erstarrt: Schmelzpunkt (aus Methylcyclohexan) 40 bis 43°C.

b) 0,4 Mol 5-Methyl-4-isoxazolcarbonsäurechlorid (58,2 g), emulgiert in 300 ml Wasser, werden tropfenweise mit 0,4 Mol Piperidin (34,1 g) und 40 ml 10 N Natronlauge so versetzt, daß die Temperatur der Reaktionsmischung nicht über 35°C steigt. Anschließend versetzt man mit 40 ml 2 N Natronlauge und extrahiert mit 300 ml Methylenchlorid. Die Wasserphase wird nochmals mit 200 ml Methylenchlorid ausgeschüttelt. Die vereinigten Methylenchloridphasen werden mit Wasser gewaschen und nach dem Trocknen über Natriumsulfat unter vermindertem Druck zur Trockne gebracht. Man erhält so N-(5-Methyl-4-isoxazolylcarbonyl)-piperidin, das nach Umkristallisation aus Methylcyclohexan einen Schmelzpunkt von 40 bis 43°C besitzt.

2) <u>N-(5-Äthyl-4-isoxazolylcarbonyl)-piperidin</u>

0,1 Mol 5-Äthyl-4-isoxazolcarbonsäurechlorid (16,0 g) gelöst in 200 ml Acetonitril werden unter Rühren bei Raumtemperatur tropfenweise mit 0,2 Mol Piperidin (17,0 g) versetzt. Man läßt 20 Minuten nachrühren, kühlt dann auf Raumtemperatur ab und saugt vom ausgefallenen Piperidinhydrochlorid ab. Das Filtrat wird unter vermindertem Druck eingeengt. Es wird ein öliger Rückstand erhalten, den man in 200 ml Methylenchlorid aufnimmt und zunächst mit 100 ml 2 N Salzsäure und anschließend mit Wasser wäscht. Nach dem Trocknen über Natriumsulfat wird unter vermindertem Druck eingeengt und der ölige Rückstand unter vermindertem Druck destilliert (Kugelrohrdestillation). Man erhält so N-(5-Äthyl-4-isoxazolylcarbonyl)-piperidin vom Siedepunkt (7 mbar) 90 - 110°C.

3) <u>N-(5-Methyl-4-isoxazolylcarbonyl)-pyrrolidin</u>

0,15 Mol 5-Methyl-4-isoxazolcarbonsäurechlorid (21,8 g), gelöst in 50 ml Acetonitril, werden unter Rühren tropfenweise mit 0,3 Mol Pyrrolidin (21,3 g) so versetzt, daß die Temperatur der Reaktionslösung nicht über 30°C steigt.

Nach 1,5-stündigem Nachrühren bei Raumtemperatur wird die Reaktionslösung unter vermindertem Druck

eingeengt, der ölige Rückstand in 200 ml Methylenchlorid gelöst und mit 30 ml 0,1 N Salzsäure geschüttelt. Nach Waschen der organischen Phase mit Wasser wird über Natriumsulfat getrocknet und unter vermindertem Druck zur Trockne eingedampft.

Man erhält so N-(5-Methyl-4-isoxazolylcarbonyl)-pyrrolidin, das sich aus Petrolether umkristallisieren läßt und einen Schmelzpunkt von 51 bis 53°C zeigt.

Analog den voranstehend beschriebenen Beispielen werden die folgenden Verbindungen der Formel I dargestellt:

4) 4-Methyl-1-(5-methyl-4-isoxazolylcarbonyl)-piperidin

vom Schmelzpunkt 44 bis 45°C, hergestellt aus 5-Methyl-4-isoxazolcarbonsäurechlorid und 4-Methylpiperidin.

5) N-(5-Methyl-4-isoxazolylcarbonyl)-thiomorpholin

vom Schmelzpunkt 64,5 bis 65°C, hergestellt aus 5-Methyl-4-isoxazolcarbonsäurechlorid und Thiomorpholin.

6) 4-Hyrdroxy-1-(5-methyl-4-isoxazolylcarbonyl)-piperidin

vom Schmelzpunkt 70,5 bis 72,5°C, hergestellt aus 5-Methyl-4-isoxazolcarbonsäurechlorid und 4-Hydroxypiperidin.

7) N-(4-Isoxazolylcarbonyl)-piperidin

vom Schmelzpunkt 78 bis 81°C, hergestellt aus 4-Isoxazolcarbonsäurechlorid und Piperidin.

8) N-(5-Trifluormethyl-4-isoxazolylcarbonyl)-piperidin

vom Siedepunkt (4 mbar) 62 bis 68°C, hergestellt aus 5-Trifluormethyl-4-isoxazolcarbonsäurechlorid und Piperidin.

9) N-(5-propyl-4-isoxazolylcarbonyl)-piperidin

vom Siedepunkt (4 mbar) 54 bis 92°C, hergestellt aus 5-Propyl-4-isoxazolcarbonsäurechlorid und Piperidin.

10) N-(5-Methyl-4-isoxazolylcarbonyl)-morpholin

vom Schmelzpunkt 42 bis 44°C, hergestellt aus 5-Methyl-4-isoxazolcarbonsäurechlorid und Morpholin.

**Pharmakologische Prüfung und Ergebnisse**

1. Analgetische Wirkung

a) Essigsäure-strecktest an der Maus nach R. Koster et al., Fed. Prod. 18, 412 (1959)

Als Versuchstiere dienten weibliche Mäuse eines N.M.R.I.-Stammes mit einem Körpergewicht (KG) zwischen 21 und 28 g. Gruppen von je 12 Tieren erhielten 0,1 ml/10 g KG einer 0,6 %-igen Essigsäurelösung intraperitoneal injiziert. Die Prüfsubstanzen wurden 30 min vorher verabreicht. Unmittelbar nach der Essigsäure-Injektion wurden die Tiere einzeln gesetzt und die innerhalb 15 min auftretenden typischen Streckbewegungen gezählt, die in einem kurzen Anspannen der Bauchmuskulatur mit Einziehen der Flankenpartien und anschließendem Strecken des Hinterkörpers und mindestens einer Hinterextremität bestehen.

Zur Beurteilung des analgetischen Effektes wurde die Anzahl der Streck-Reaktionen in Beziehung zu jener einer unbehandelten Kontrollgruppe gesetzt, wobeijene Tiere, die weniger als die Hälfte der durchschnittlich von den Kontrolltieren ausgeführten Streckbewegungen zeigten, als analgesiert gewertet wurden.

Die Prüfsubstanzen wurden oral in einem Volumen von 10 ml/kg KG in 1 %-iger wäßriger Carboxymethylcellulose (CMC)-Suspension appliziert.

b) Modifizierter Randall-Selitto-Hyperalgesietest an der Ratte nach Atkinson et al., J. Pharm. Pharmac. 26, 727 (1974)

Als Versuchstiere wurden männliche Sprague-Dawley-Ratten mit einem Körpergewicht von 200 bis 300 g verwendet. Unter leichter Äthernarkose wurde den Versuchstieren subplantar in die linke Hinterpfote 0,2 ml Bierhefe-Suspension (40 % Hefe in 0,9 % NaCl-Lösung) injiziert. Nach fünf Stunden wurde der Gang der Tiere über einen Metall-Gitter-Rost nach folgendem Schema beurteilt:

0 = dreibeiniger Gang
0,5 = starkes Hinken
1 = normaler Gang

Die Auswertung erfolgte durch Bestimmung des prozentualen Anteils an Tieren, die mit Stufe 1 bzw. 0,5 beurteilt wurden, wobei zwei Tiere, die 0,5 erhielten, als ein analgesiertes gewertet wurden.

Die Prüfsubstanzen wurden oral an 15 Stunden nüchternen Tieren in CMC-Suspension in einem Volumen von 10 ml/kg KG zwei Stunden vor Beurteilung der Tiere verabreicht. Um subjektive Einfüsse auszuschließen, wurde die Beurteilung von zwei Personen unabhängig voneinander und ohne Wissen um die Vorbehandlung der Tiere durchgeführt (n = 10/dosi). Die $ED_{50}$-Werte wurden mit Hilfe der linaren Regression nach Fieller und Sidak bestimmt.

2. <u>Antipyretische Wirkung</u>

Die Untersuchungen wurden an mit Trinkwasser ad libitum und Standarddiät ernährten weiblichen Sprague-Dawley-Ratten mit einem Körpergewicht von 150 g durchgeführt. Die erhöhte Körpertemperatur wurde durch subcutane Injektion von 10 ml/kg 15 %-iger (Gew./Vol.) Bierhefesuspension in 0,9 %-iger NaCl-Lösung induziert, worauf den Tieren das Futter bis zum Versuchsende entzogen wurde. 18 Stunden nach Hefeinjektion erfolgte die orale Verabreichung der Testsubstanzen in CMC-Suspension in einem Volumen von 10 ml/kg KG. Die Messung der Körpertemperatur wurde rektal mittels Sekundenthermometer bei Raumtemperatur (24°C) vorgenommen. Die Anzahl der Tiere betrug n = 6 pro dosi. Registriert wurde die mittlere Senkung der Körpertemperatur im Vergleich zu dem gleichzeitigen Wert der unbehandelten Kontrolltiere.

3. <u>Gastrointestinale Ulcerogenität</u>

Bei diesem Test an männlichen Sprague-Dawley-Ratten mit einem Körpergewicht zwischen 200 und 300 g wurde durch Hunger-Streß (Futterentzug für insgesamt 72 Stunden) eine erhöhte Sensibilität der gastralen Mucosa gegenüber der ulcerogenen Wirkung von nichtsteroidalen Antiphlogistika induziert.

48 Stunden vor Applikation der Testsubstanzen wurde den Tieren bei freiem Zugang zum Trinkwasser das Futter bis zum Versuchsende entzogen.

24 Stunden nach der oralen Präparatgabe erfolgte die Tötung und Entnahme der Mägen, die entlang der kleinen Kurvatur aufgeschnitten, unter fließendem Wasser gereinigt und auf Schleimhautläsionen inspiziert wurden. Als Ulcera galten alle makroskopisch sichtbaren Läsionen der Mucosa im Drüsenmagen. Bestimmt wurde der Anteil der Tiere mit Ulcera pro dosi.

Die Testsubstanzen wurden, gelöst in CMC-Suspension, in einem Volumen von 1 ml/100 g Körpergewicht verabreicht. Die $UD_{50}$ (Dosis, bei der 50 % der Tiere Ulcera aufweisen) wurde mit Hilfe der Probit-Analyse und der Vertrauensbereich nach Fieller bestimmt.

4. <u>Akute Toxizität</u>

Die $LD_{50}$-Werte wurden nach oraler Applikation der prüfpräparate an männlichen und weiblichen Wistar-Ratten mit einem Körpergewicht von 130 - 150 g nach Litchfield und Wilcoxon ermittelt. Die Tiere kamen etwa 18 stunden nüchtern zum Versuch und bekamen erst fünf Stunden nach präparatgabe wieder Futter. Nach dreiwöchiger Beobachtungszeit wurdan sie mit Chloroform getötet und seziert. Es erfolgte eine makroskopische Begutachtung der Organe. Die Testverbindungen wurden in 1 %-iger CMC-suspension gelöst und den Ratten in einem Volumen von 5 ml/kg KG mit der Schlundsonde verabreicht.

5. <u>Ergebnisse</u>

Die erfindungsgemäßen Verbindungen der Formel I zeigen im Strecktest an der Maus starke analgetische Wirksamkeit, die in ihrem Ausmaß den beiden Standardanalgetika Paracetamol und Acetylsalicylsäure überlegen oder annähernd gleichwertig ist (Tabelle 1). Die Acetylsalicylsäure wurde auf Grund ihrer generellen Bedeutung als Analgetikum zusätzlich als Vergleichssubstanz herangezogen, obwohl sie von ihrem Wirkungsmechanismus als Inhibitor der peripheren Prostaglandin-Biosynthese und damit antiphlogistisch wirksames Analgetikum her - wie eingangs dargelegt - von den erfindungsgemäßen Verbindungen zu unterscheiden ist. Die beiden aus der Deutschen Patentschrift 634 286 bekannten Isoxazolverbindungen liegen mit einer Hemmung von 25 % im Streubereich der Methode und sind somit analgetisch unwirksam.

**Tabelle 1:** Analgetische Wirkung im Essigsäure-Strecktest an der Maus

| Verbindung des Beispiels | Analgesierte Tiere in % nach einer oralen Dosis von 155 mg/kg |
|---|---|
| 1 | 83 |
| 2 | 83 |
| 3 | 83 |
| 4 | 67 |
| 5 | 59 |
| 6 | 50 |
| 7 | 50 |
| 8 | 75 |
| 9 | 67 |
| 10 | 42 |
| 3-,5-Dimethyl-4-isoxazolcarbonsäurepiperidid *) | 25 |
| 5-Methyl-4-isoxazolcarbonsäure-N,N-diäthylamid **) | 25 |
| Paracetamol | 50 |
| Acetylsalicylsäure | 48 |

*) Deutsche Patentschrift 634 286, Beispiel 6
**) Deutsche Patentschrift 634 266, Beispiel 5 (dort keine Zuordnung der Methylgruppe)

Auch in den anderen Testmodellen ließ sich die Überlegenheit der erfindungsgemäßen Verbindungen gegenüber den Standardpräparaten klar demonstrieren (Tabelle 2). So weist beispielsweise die Verbindung des Beispiels in dem spezifischen Schmerzmodell nach Randall-Selitto eine deutlich günstigere mittlere Wirkdosis ($ED_{50}$) auf. Sie ist mit 85 mg/kg doppelt so aktiv wie Acetylsalicylsäure und viermal so stark wirksam wie Paracetamol. Auch im Schmerztest an der Maus hat sie mit einer $ED_{50}$ von 45 mg/kg per os mindestens die dreifache Wirkung wie die beiden Vergleichspräparate.

Ihre aus der Relation zur Toxizität sich ergebende therapeutische Breite ($LD_{50}$ : $E_{50}$) liegt ebenfalls in beiden schmerztests etwa zwei- bis dreimal so günstig wie jene der beiden Vergleichspräparate.

Für die Beurteilung der therapeutischen Unbedenklichkeit von Analgetika ist vor allem auch die gastrale Ulcerogenität entscheidend. Hier zeigt beispielsweise die Verbindung des Beispiels 1 eine hervorragende Verträglichkeit, da in Dosen bis zu 400 mg/kg keine Schleimhautläsionen nachgewiesen wurden. Acetylsalicylsäure weist eine mittlere ulcerogene Dosis ($UD_{50}$) von 31 mg/kg auf und reflektiert damit die auch bei der Anwendung am Menschen häufigste Nebenwirkung.

Wie bereits eingangs erwähnt, besitzen die erfindungsgemäßen Verbindungen der Formel I auch starke antipyretische Eigenschaften. Im Hefefieber-Test an der Ratte (Tabelle 3) zeigt beispielsweise die Verbindung des Beispiels 1 mit einer oralen Dosis von 50 mg/kg eine sowohl stärkere als auch deutlich länger anhaltende Fiebersenkung als das Vergleichspräparat Paracetamol mit der doppelten Dosis von 100 mg/kg per os.

**Tabelle 2:** Analgetische Wirkung, Toxizität, Ulcerogenität und therapeutische Breite

| Test-substanz | Analgetische Wirkung $ED_{50}$ in mg/kg per os | | Toxizität $LD_{50}$ in mg/kg | Ulcerogenität $UD_{50}$ in mg/kg | Therapeutische Breite $LD_{50}$ : $ED_{50}$ | |
|---|---|---|---|---|---|---|
| | Randall-Selitto-Test | Essigsäure-Strecktest | per os | per os | Randall-Selitto-Test | Essigsäure-Strecktest |
| | (Ratte) | (Maus) | (Ratte) | (Ratte) | | |
| Acetylsalicylsäure | 182 | 138 | 1 500 | 31 | 8,2 | 10,9 |
| Paracetamol | 334 | 155 | 1 944 | >400 | 5,8 | 12,5 |
| Beispiel 1 | 85 | 45 | 1 240 | >400 | 14,6 | 27,6 |

**Tabelle 3:** Antipyretische Wirkung an Ratten

| Test-substanz | Dosis in mg/kg per os | Senkung der Körpertemperatur in °C zur Zeit t (in Minuten) | | | |
|---|---|---|---|---|---|
| | | 60 | 120 | 180 | 240 min |
| Paracetamol | 100 | 1,3 | 1,1 | 0,4 | 0 |
| Beispiel 1 | 50 | 1,3 | 1,6 | 1,3 | 0,8 |
| Beispiel 1 | 100 | 2,0 | 2,0 | 1,5 | 1,1 |

Aus der Literatur ist bekannt, daß sich Paracetamol wegen des Fehlens einer spasmolytischen Wirkungskomponente für die Behandlung spastisch bedingter Schmerzen der Gallenwege, des Gastrointestinal- und Urogenitaltraktes nicht eignet (R. K. Liedtke, Medizinische Klinik 77 (1982), Seiten 34 - 40). Im Einklang hiermit zeigt Paracetamol keine Hemmwirkung auf den am isolierten Schweine-Ureter beispielsweise mit Kaliumchlorid ausgelösten Spasmus. Im Gegensatz dazu üben die Verbindungen der Formel I in dieser Versuchsanordnung eine signifikante Hemmwirkung aus. So liegt beispielsweise der $ED_{50}$-Wert für die Verbindung des Beispiels 1 bei einer Konzentration von 410 µg/ml.

Die erfindungsgemäßen Verbindungen besitzen folglich den großen Vorzug gegenüber Paracetamol, daß sie auch zur Therapie von Schmerzzuständen spastischer Genese eingesetzt werden können.

Ein weiterer Vorteil ist schließlich in der Wasserlöslichkeit der erfindungsgemäßen Verbindungen zu sehen, die die Herstellung parenteral verabreichbarer Zubereitungen gestattet.

**Patentansprüche** für die Vertragsstate: BE, CH, DE, FR, GB, IT LI, LN, NL, SE

1. 4-Isoxazoicarbonsäureamide der Formel I,

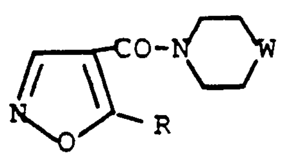

(I)

in der

R Wasserstoff, Alkyl mit bis zu 4 C-Atomen oder Halogenalkyl mit bis zu 2 C-Atomen und
W eine direkte Bindung, $CH_2$, $CH-CH_3$, $CH-C_2H_5$, CHOH, O oder S bedeuten.

2. Verbindungen gemäß Anspruch 1, dadurch gekennzeichnet, daß R einen Methyl-, Äthyl oder Trifluormethyl-Rest darstellt.

3. Verbindungen nach Anspruch 2, dadurch gekennzeichnet, daß W $CH_2$ bedeutet.

4. Verbindung nach Anspruch 1, dadurch gekennzeichnet, daß R einen Methylrest darstellt und W $CH_2$ bedeutet.

5. Verfahren zur Herstellung von verbindungen der Formel I gemäß Anspruch 1, dadurch gekennzeichnet, daß man

a) ein Amin der Formel II

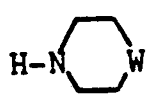

(II)

mit einem 4-Isoxazolcarbonsäure-Derivat der Formel III,

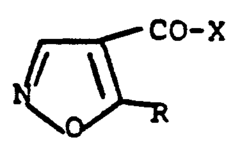

(III)

in der X entweder ein Halogenatom oder ein YO- oder ZO-CO-O-Gruppe bedeutet, wobei Y für Phenyl, durch Fluor, Chlor, Brom, Jod, Methyl, Äthyl, Methoxy, Äthoxy, Trifluormethyl, Nitro oder Cyan einfach, zweifach oder dreifach substituiertes phenyl oder für den Acylrest der Formel IIIa,

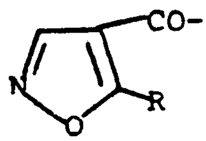

(IIIa)

und Z für ($C_1$-$C_4$)-Alkyl, Phenyl oder Benzyl stehen, umsetzt oder

$b_1$) ein 2-Acylessigsäureamid der Formel IV,

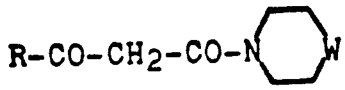

(IV)

mit einem Orthoameisensäureester der Formel $HC(OR^1)_3$ (V), worin $R^1$ ($C_1$-$C_4$)Alkyl bedeutet, umsetzt und das dabei erhaltene 2-Alkoxymethylen-2-acylessigsäureamid der Formel VI

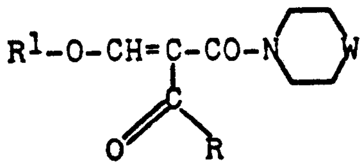

(VI)

mit Hydroxylamin zum Produkt der Formel I cyclisiert oder

$b_2$) das 2-Acylessigsäureamid der Formel IV mit sekundären Aminen der Formel $HNR^2R^3$ (VII), in der $R^2$ und $R^3$ gleiche oder verschiedene Alkylrestemit 1 bis 4 C-Atomen bedeuten oder zusammen mit dem Stickstoffatom, an das sie gebunden sind, den Pyrrolidin-, Piperidin- oder Morpholinring bilden, zu einem Enamin der Formel VIII,

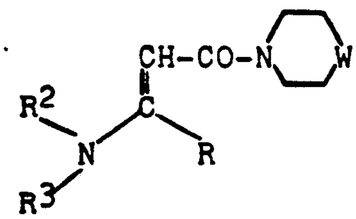

(VIII)

umsetzt und dieses mit Nitromethan in Gegenwart wasserentziehender Mittel zum Isoxazol-Derivat der Formel I cyclisiert, wobei in den Formeln II, III, IIIa, IV, VI und VIII R und w die im Anspruch 1 definierten Reste darstellen.

6. Arzneimittel, dadurch gekennzeichnet, daß sie Verbindungen der Formel I gemäß einem oder mehreren der Ansprüche 1 bis 4 enthalten.

7. Verbindungen gemäß einem oder mehreren der Ansprüche bis 4 zur Verwendung bei der Behendlung von Schmerz- und/oder Fieberzuständen.

**Patentansprüche** für der Vertragsstat: AT

1. Verfahren zur Herstellung von 4-Isoxazolcarbonsäureamiden der Formel I,

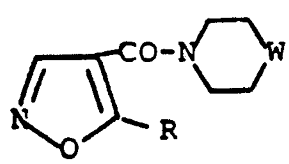

(I)

in der

R Wasserstoff, Alkyl mit bis zu 4 C-Atomen oder Halogenalkyl mit bis zu 2 C-Atomen und
W eine direkte Bindung, $CH_2$, $CH$-$CH_3$, $CH$-$C_2H_5$, CHOH, O oder S bedeuten,
dadurch gekennzeichnet, daß man

a) ein Amin der Formel

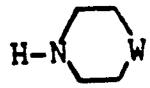

(II)

mit einem 4-Isoxazolcarbonsäure-Derivat der Formel III,

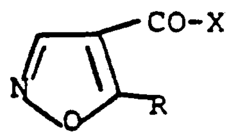

(III)

in der X entweder ein Halogenatom oder ein YO- oder ZO-CO-O-Gruppe bedeutet, wobei Y für Phenyl, durch Fluor, Chlor, Brom, Jod, Methyl, Äthyl, Methoxy, Äthoxy, Trifluormethyl, Nitro oder Cyan einfach, zweifach oder dreifach substituiertes Phenyl oder für den Acylrest der Formel IIIa,

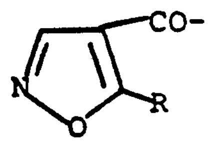

(IIIa)

und Z für ($C_1$-$C_4$)-Alkyl, Phenyl oder Benzyl stehen, umsetzt oder

$b_1$) ein 2-Acylessigsäureamid der Formel IV,

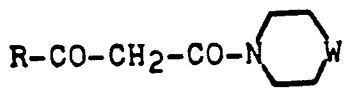

(IV)

mit einem Orthoameisensäureester der Formel $HC(OR^1)_3$ (V), worin $R^1$ ($C_1$-$C_4$)Alkyl bedeutet, umsetzt und das dabei erhaltene 2-Alkoxymethylen-2-acylessigsäureamid der Formel VI

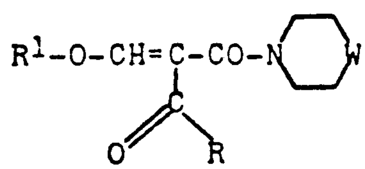

(VI)

mit Hydroxylamin zum Produkt der Formel I cyclisiert oder

$b_2$) das 2-Acylessigsäureamid der Formel IV mit sekundäre, Aminen der Formel $HNR^2R^3$ (VII), in der $R^2$ und $R^3$ gleiche oder verschiedene Alkylrestemit 1 bis 4 C-atomen bedeuten oder zusamme, mit dem Stickstoffatom, an das sie gebunden sind, den Pyrrolidin-, Piperidin- oder Morpholinring bilden, zu einem Enamin der Formel VIII,

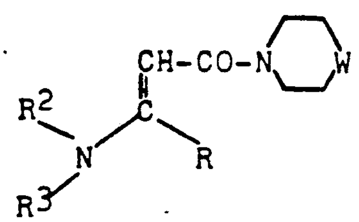

(VIII)

umsetzt und dieses mit Nitromethan in Gegenwart wasserentziehender Mittel zum Isoxazol-Derivat der Formel cyclisiert, wobei in den Formeln II, III, IIIa, IV, VI und VIII R und W die oben definierten Reste darstellen.

2. Verfahren gemäß Anspruch 1, dadurch gekennzeichnet, daß R einen Methyl-, Äthyl- oder Trifluormethyl-Rest darstellt.

3. Verfahren nach Anspruch 2, dadurch gekennzeichnet, daß W $CH_2$ bedeutet.

4. Verfahren nach Anspruch 1, dadurch gekennzeichnet, daß R einen Methylrest darstellt und W $CH_2$ bedeutet.

**Claims** for the contracting states: BE, CH, DE, FR, GB, IT, LI, LU, NL SE

1. A 4-isoxazolecarboxylic acid amide of the formula I

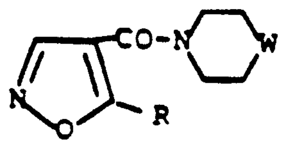

(I)

in which

R denotes hydrogen, alkyl with up to 4 carbon atoms or halogenoalkyl with up to 2 carbon atoms and W denotes a direct bond, $CH_2$, $CH\text{-}CH_3$, $CH\text{-}C_2H_5$, CHOH, O or S.

2. A compound as claimed in claim 1, characterized in that R represents a methyl, ethyl or trifluoromethyl radical.

3. A compound as claimed in claim 2, characterized in that W denotes $CH_2$.

4. The compound as claimed in claim 1, characterized in that R represents a methyl radical and W denotes $CH_2$.

5. A process for the preparation of a compound of the formula I as claimed in claim 1, characterized by

a) reacting an amine of the formula II

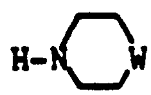

(II)

with a 4-isoxazolecarboxylic acid derivative of the formula III

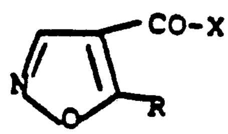

(III)

in which X denotes either a halogen atom or a YO- or ZO-CO-O- group, in which Y represents phenyl, phenyl which is mono-, di- or tri-substituted by fluorine, chlorine, bromine, iodine, methyl, ethyl, methoxy, ethoxy, trifluoromethyl, nitro or cyano, or the acyl radical of the formula IIIa

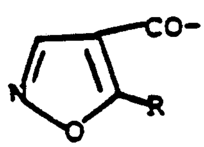

(IIIa)

and Z represents ($C_1$-$C_4$)-alkyl, phenyl or benzyl, or $b_1$) reacting a 2-acylacetic acid amide of the formula IV

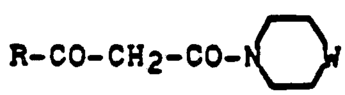

(IV)

with an orthoformic acid ester of the formula $HC(OR^1)_3$ (V), in which $R^1$ denotes ($C_1$-$C_4$)alkyl, and cyclizing the 2-alkoxymethylene-2-acylacetic acid amide thereby obtained of the formula VI

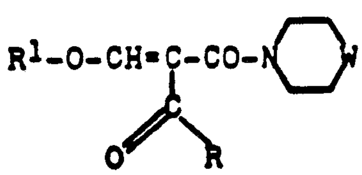

(VI)

with hydroxylamine to give the product of the formula I, or

$b_2$) reacting the 2-acylacetamide of the formula IV with a secondary amine of the formula $HNR^2R^3$ (VII), in which $R^2$ and $R^3$ denote identical or different alkyl radicals with 1 to 4 carbon atoms or, together with the nitrogen atom to which they are bonded, form the pyrrolidine, piperidine or morpholine ring, to give an enamine of the formula VIII

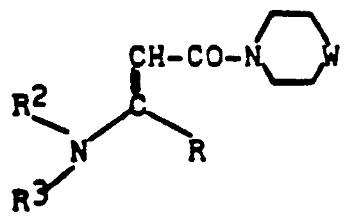

(VIII)

and cyclizing this with nitromethane in the presence of a dehydrating agent to give the isoxazole derivative of the formula I, R and W in the formulae II, III, IIIa, IV, VI and VIII representing the radicals defined in claim 1.

6. Medicaments, characterized in that they contain compounds of the formula I as claimed in any one of claims 1 to 4.

7. A compound as claimed in any one of claims 1 to 4 for the treatment of pain and/or fever.

**Claims** for the contracting state: AT

1. A process for the preparation of a 4-isoxazolecarboxylic acid amide of the formula I

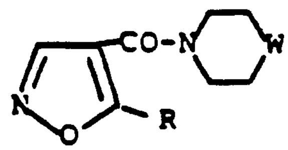

(I)

in which

R denotes hydrogen, alkyl with up to 4 carbon atoms or halogenoalkyl with up to 2 carbon atoms and
W denotes a direct bond, $CH_2$, $CH\text{-}CH_3$, $CH\text{-}C_2H_5$, CHOH, O or S,
characterized by
a) reacting an amine of the formula II

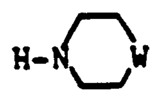

(II)

with a 4-isoxazolecarboxylic acid derivative of the formula III

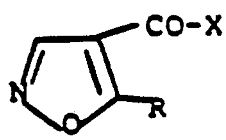

(III)

in which X denotes either a halogen atom or a YO- or ZO-CO-O- group, in which Y represents phenyl, phenyl which is mono-, di- or tri-substituted by fluorine, chlorine, bromine, iodine, methyl, ethyl, methoxy, ethoxy, trifluoromethyl, nitro or cyano, or the acyl radical of the formula IIIa

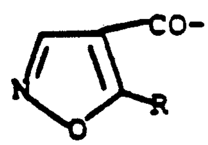

(IIIa)

and Z represents ($C_1$-$C_4$)-alkyl phenyl or benzyl, or

$b_1$) reacting a 2-acylacetic acid amide of the formula IV

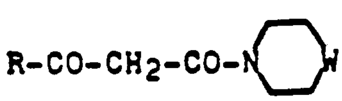

(IV)

with an orthoformic acid ester of the formula $HC(OR^1)_3$ (V), in which $R^1$ denotes ($C_1$-$C_4$)alkyl, and cyclizing the 2-alkoxymethylene-2-acylacetic acid amide thereby obtained of the formula VI

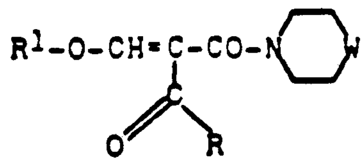

(VI)

with hydroxylamine to give the product of the formula I, or

$b_2$) reacting the 2-acylacetamide of the formula IV with a secondary amine of the formula $HNR^2R^3$ (VII), in which $R^2$ and $R^3$ denote identical or different alkyl radicals with 1 to 4 carbon atoms or, together with the nitrogen atom to which they are bonded, form the pyrrolidine, piperidine or morpholine ring, to give an enamine of the formula VIII

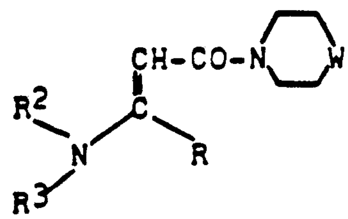

(VIII)

and cyclizing this with nitromethane in the presence of a dehydrating agent to give the isoxazole derivative of the formula I, R and W in the formulae II, III, IIIa, IV, VI and VIII representing the radicals defined above.

2. The process as claimed in claim 1, characterized in that R represents a methyl, ethyl or trifluoromethyl radical.

3. The process as claimed in claim 2, characterized in that W denotes $CH_2$.

4. The process as claimed in claim 1, characterized in that R represents a methyl radical and W denotes $CH_2$.

**Revendications** pour les états contractants: BE, CH, DE, FR, GB, IT, LI, LU, NL, SE

1. 4-isoxazolcarboxamides de formule I

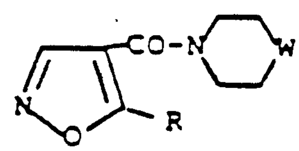

(I)

dans laquelle

R représente l'hydrogène, un groupe alcoyle ayant jusqu'à 4 atomes de carbone ou halogénoalcoyle ayant jusqu'à 2 atomes de carbone et

W représente une liaison directe, $CH_2$, $CH\text{-}CH_3$, $CH\text{-}C_2H_5$, CHOH, O ou S.

2. Composés selon la revendication 1, caractérisés en ce que R représente un groupe méthyle, éthyle ou trifluorométhyle.

3. Composés selon la revendication 2, caractérisés en ce que W représente $CH_2$.

4. Composés selon la revendication 1, caractérisés en ce que R représente un groupe méthyle et W est $CH_2$.

5. Procédé pour la préparation de composés de formule I selon la revendication 1, caractérisé en ce que:

a) on fait réagir une amine de formule II

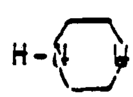

(II)

avec un dérivé de l'acide 4-isoxazolcarboxylique de formule III

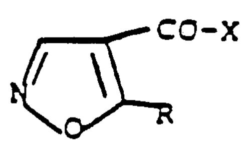

(III)

dans laquelle X représente un atome d'halogène ou un groupe YO ou ZO-CO-O, Y étant un groupe phényle, un groupe phényle de 1 à 3 fois substitué par le fluor, le chlore, le brome, l'iode, un groupe méthyle, éthyle, méthoxy, éthoxy, trifluorométhyle, nitro ou cyano, ou le groupe acyle de formule IIIa

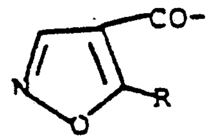

(IIIa)

et Z représente un groupe alcoyle en $C_1$-$C_4$, phényle ou benzyle ou

$b_1$) on fait réagir un 2-acylacétamide de formule IV

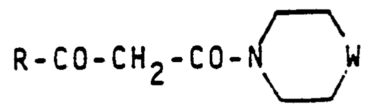

(IV)

avec un ester de l'acide orthoformique de formule $HC(OR^1)_3$ (V) dans lequel $R^1$ représente un groupe alcoyle en $C_1$-$C_4$, et on cyclise le 2-alcoxyméthylène-2-acylacétamide de formule VI ainsi obtenu

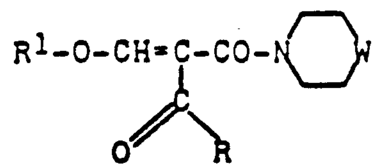

(VI)

avec de l'hydroxylamine en un produit de formule I, ou

$b_2$) on fait réagir le 2-acylacétamide de formule IV avec des amines secondaires de formule $HNR^2R^3$ (VII) dans laquelle $R^2$ et $R^3$ représentent des groupes alcoyle en $C_1$-$C_4$ identiques ou différents, ou forment ensemble avec l'atome d'azote sur lequel ils sont fixés, un noyau pyrrolidine, pipéridine ou morpholine, pour obtenir une énamide de formule VIII

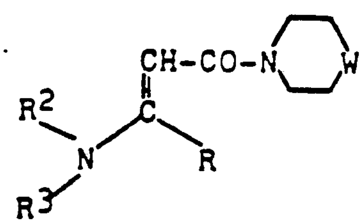

(VIII)

et on cyclise cette dernière avec du nitrométhane en présence d'un accepteur d'eau en un dérivé d'isoxazole de formule I, R et W dans les formules II, III, IIIa, IV et VI et VIII représentant les groupes tels que définis sous 1.

6. Médicaments caractérisés en ce qu'ils contiennent des composés de formule I selon l'une quelconque des revendications 1 à 4.

7. Utilisation des composés selon l'une quelconque des revendications 1 à 4 pour le traitement des états douloureux ou fébriles.

**Revendications** pour l'état contractant: AT

1. Procédé de préparation de 4-isoxazolcarboxamides de formule I

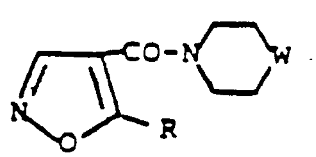

(I)

dans laquelle

R représente l'hydrogène, un groupe alcoyle ayant jusqu'à 4 atomes de carbone ou halogénoalcoyle ayant jusqu'à 2 atomes de carbone et

W représente une liaison directe, $CH_2$, $CH$-$CH_3$, $CH$-$C_2H_5$, CHOH, O ou S, caractérisé en ce que:

a) on fait réagir une amine de formule II

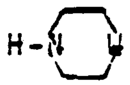

(II)

avec un dérivé de l'acide 4-isoxazolcarboxylique de formule III

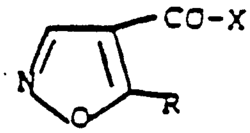

(III)

dans laquelle X représente un atome d'halogène ou un groupe YO ou ZO-CO-O, Y étant un groupe phényle, un groupe phényle de 1 à 3 fois substitué par le fluor, le chlore, le brome, l'iode, un groupe méthyle, éthyle, méthoxy, éthoxy, trifluorométhyle, nitro ou cyano, ou le groupe acyle de formule IIIa

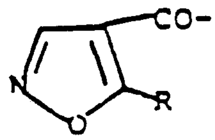

(IIIa)

et z représente un groupe alcoyle en $C_1$-$C_4$, phényle ou benzyle ou

$b_1$) on fait réagir un 2-acylacétamide de formule IV

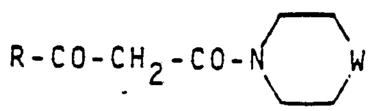

(IV)

avec un ester de l'acide orthoformique de formule HC $(OR^1)_3$ (V) dans lequel $R^1$ représente un groupe alcoyle en $C_1$-$C_4$, et on cyclise le 2-alcoxyméthylène-2-acylacétamide de formule VI ainsi obtenu

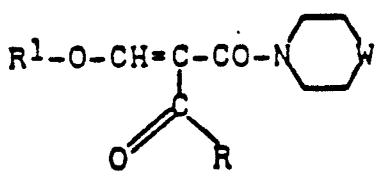

(VI)

avec de l'hydroxylamine en un produit de formule I, ou

$b_2$) on fait réagir le 2-acylacétamide de formule IV avec des amides secondaires de formule $HNR^2R^3$ (VII) dans laquelle $R^2$ et $R^3$ représentent des groupes alcoyle en $C_1$-$C_4$ indentiques ou différents, ou forment ensemble avec l'atome d'azote sur lequel ils sont fixés, un noyau pyrrolidine, pipéridine ou morpholine, pour obtenir une énamide de formule VIII

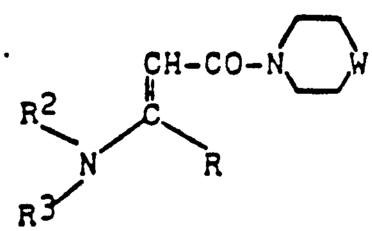

(VIII)

et on cyclise cette dernière avec du nitrométhane en présence d'un accepteur d'eau en un dérivé d'isoxazole de formule I, R et W dans les formules II, III, IIIa, IV, VI et VIII représentant les groupes tels que définis ci-dessus.

2. Procédé selon la revendication 1, caractérisé en ce que R représente un groupe méthyle, éthyle ou trifluorométhyle.

3. Procédé selon la revendication 2, caractérisé en ce que W représente $CH_2$.

4. Procédé selon la revendication 1, caractérisé en ce que R représente un groupe méthyle et W est $CH_2$.